# EUROPEAN PATENT SPECIFICATION

(11) **EP 0 820 781 B1**
(45) Date of publication and mention of the grant of the patent: **21.01.2004**
(21) Application number: 97202284.2
(22) Date of filing: 22.07.1997
(51) Int. Cl.: A61M 25/00, B65D 85/04, B65H 75/40

(54) **Packaging support for tube-like elements with varying lengths**
Träger zum Verpacken von rohrförmigen Elementen mit variierenden Längen
Support d'emballage pour éléments tubulaires de longueurs variables

(30) Priority: 22.07.1996 NL 1003656
(43) Date of publication of application: 28.01.1998
(73) Proprietor: CORDIS EUROPA N.V., NL-9301 LJ Roden (NL)
(72) Inventor: Van Es, Bert, 9301 CD Roden (NL)
(74) Representative: 't Jong, Bastiaan Jacob

(56) References cited:
- DE-A- 4 333 073
- DE-U- 8 437 873

## Description

The invention relates to a packaging support for tube-like elements such as catheters.

It is known to package catheters using a packaging support which consists of an elongated plate onto which the catheter is fixed in an extended manner. The packaging support, with the catheter fixed onto it, is then inserted into an elongated envelope, after which the envelope is sealed.

Such known packaging supports have to be manufactured and kept in store in an assortment of sizes as catheters may be offered in a great variety of lengths.

DE 84 37 873 U discloses an implantable catheter comprising a port, with in it's interior space a reel around which the catheter tubing is placed. The catheter tubing can be extracted from the interior of the port space in a desired length.

The object of the invention is to provide a packaging support for tube-like elements such as catheters, which can be used universally.

This aim is achieved with the packaging support according to the invention as characterised in claim 1. The tube-like element is secured at at least one end by the securing means and is furthermore wound around the standing wall of the raised section of the packaging support and the ends are secured by the securing means.

With the packaging support according to the invention, tube-like elements with a large variety of lengths can be packaged, so that only one type of packaging support can be used for all these types of tube-like elements.

The enclosing elements prevent the tube-like element from moving off the standing wall.

By rotating the raised section, the securing means connected to the raised section can be placed in the correct position, so that the entire length of the tube-like element can be wound carefully around the standing wall without slack, whereby the ends are kept accurately in position by the securing means. As the securing means can be turned into the correct position, just one securing means on the raised section will suffice.

With the packaging support according to the invention, no separate connecting means are required in order to connect the raised section in a rotatable manner to the base plate. For this purpose the standing wall of the raised section has a circular basic shape and has been received in a corresponding circular opening of the base plate.

The raised section of the packaging support according to the invention comprises a flange at the end of the standing wall situated close to the base plate. As this flange is enclosed in between the two sections of the base plate, the raised section is kept accurately in position without coming loose out of the opening in the base plate. The wound tube-like element is consequently retained in a reliable manner.

Additionally, the base plate may have been formed in a suitable manner by a plate folded double. No separate connecting means need to be used in that case in order to connect the two sections of the base plate to each other.

An economic embodiment of the packaging support according to the invention is achieved by employing the measure as set out in claim 3. The elements cut out of the standing wall can easily be punched out when forming the raised section of the packaging support.

When employing the measure as set out in claim 4 it is achieved that the entire packaging support is given a closed appearance, whereby the end of the tube-like element connected to the raised section can be secured to this flat plate section in a suitable manner.

An advantageous further development has been characterised in claim 5. The free ends of the tube-like element are properly visible in the packaging. When used for a catheter, the writing on the connecting member at the proximal end of the catheter is properly visible and legible, whereas at the same time the specific shape of the distal end can be seen properly.

The packaging support according to the invention is preferably made of cardboard. Both the base plate and the raised section can be made of sheet cardboard. The raised section can also be made according to techniques known as such from cardboard pulp, which techniques are also employed when manufacturing packaging means such as egg boxes.

Another suitable method for manufacturing the packaging support according to the invention is by blow moulding the latter from a thin sheet of plastic material. The required profiles, defined by the standing wall et cetera, can in that case be arranged accurately in the packaging support.

The invention will be explained in greater detail in the following description with reference to the attached figure.

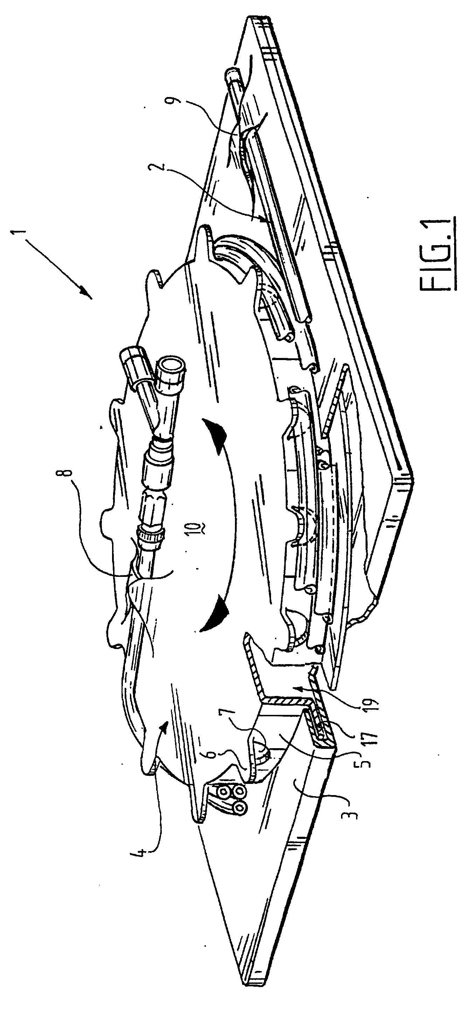

Figure 1 shows a partly broken away perspective view of a first embodiment of the packaging support according to the invention.

The packaging support illustrated in figure 1 is intended to be used for packaging catheters.

As can be seen in figure 1, the packaging support 1 has been provided with a catheter 2. The packaging support 1 comprises a base plate 3 and a raised section 4. The raised section 4 is a separate element from the base plate 3 and has a circular basic shape. This raised section 4 has been received in a corresponding circular opening 19 in the base plate 3. As a result the raised section 4 has been received in the base plate 3 in a rotatable manner.

The raised section 4 defines at its circumference an endless standing wall 5. The basic body of the catheter 2 can be wound around this standing wall.

At the end of the standing wall 5 turned away from the base plate 3, that is to say at the top as seen in figure 1, enclosing elements 6 have been formed which prevent the catheter 2 from sliding off the standing wall 5.

With the example of this embodiment the enclosing elements 6 have been formed by elements cut out of the standing wall. When manufacturing the packaging support 1, arch-shaped punch lines have been formed which define the openings 7 in the circumferential wall 5, out of which the enclosing elements 6 have been formed.

The raised section 4 has been closed off at the top by a flat plate section 10 fixed to the standing wall 5. In this flat plate section 10 securing means 8 have been formed for the proximal end of the catheter 2. With the example of the embodiment these securing means are formed by punch lines with a staggered cross-shape, as a result of which two lips are defined which secure the end of the catheter 2 in a resilient manner.

On the base plate 3 similar securing means 9 have been formed for securing the distal end of the catheter 2.

It will be clear that the packaging support 1 can receive catheters 2 with a great variety of lengths. Depending on where the proximal end of the catheter 2 finishes, one of the two securing means 8 can be used in order to secure this proximal end. As the figures illustrate, the proximal end of the catheter is bent over the upper edge of the standing wall 5 and positioned flat on top of the plate-like section 10.

In the case of the example of this embodiment the base plate 3 has been formed by a sheet folded double whereby the circular opening 19 has been formed in one of the sections folded together.

At its base the raised section 4 has been provided with a flange 17 which has been received in between the two sections Qf the base plate 3. Consequently the raised section 4 has been fixed in a rotatable manner in relation to the base plate 3.

In the upper surface of the raised section 4 one securing element 8 has been arranged. Due to the fact that the raised section 4 can be rotated, the securing element 8 can always be placed accurately in the correct position in order to secure the proximal end-section of the catheter received in the packaging, of which the ultimate position will depend on the length of this catheter.

The securing means 8, 9 have been arranged in such a position that they are situated at a distance from the standing wall. Consequently both the distal end-section of the catheter extends freely over a distance on top of the base plate and the proximal end-section over a distance on top of the upper surface of the raised section 4. Both these ends can consequently be seen very well, so that the end-user can check properly if he has obtained the required catheter.

It will be clear that after the catheter has been arranged to it, the packaging support according to figure 1 will be enclosed in a covering, which may be an envelope or a box, which will be sealed after which the whole assembly will be sterilized.

## Claims

1. Packaging support for tube-like elements such as catheters, said support comprising: a base plate (3) and a separate raised section (4) which defines at its circumference an end-less standing wall (5) with a circular basic shape around which the tube-like element (2) can be wound, said raised section (4) being connected in a rotatable manner to the base plate (3) by being received in a corresponding circular opening (19) of the base plate (3) ; enclosing elements (6) for preventing the tube-like element (2) from moving off the standing wall (5), protruding outward at the end of the standing wall (5) which is turned away from the base plate (3); and securing means (8,9) for one of the ends of the tube-like element arranged to the base plate (3) and for the other end arranged to the raised section (4), wherein the raised section (4) comprises a flange (17) at the end of the standing wall (5) situated close to the base plate (3), **characterized in that** the base plate (3) is double-layered, comprising two sections, wherein the circular opening (19) has been arranged in one of the sections thereof and the flange (17) has been received in between the two sections.

2. Packaging support as claimed in claim 1, wherein the base plate (3) is a sheet folded double.

3. Packaging support as claimed in claim 1, wherein the enclosing elements (6) have been formed by elements cut out of the standing wall.

4. Packaging support as claimed in claim 1, wherein the raised section (4) comprises a flat plate section (10) adjoining the standing wall (5).

5. Packaging support as claimed in claim 1, wherein the securing means (8,9) have been formed at a distance from the standing wall (5) in such a way that the ends of the tube-like element (2) connected to them can extend freely over a distance.

6. Packaging support as claimed in claim 1, made of cardboard.

7. Packaging support as claimed in claim 1, made of a blow-moulded thin sheet of plastic material.

## Patentansprüche

1. Träger zum Verpacken von rohrförmigen Elementen, wie beispielsweise Kathetern, wobei der Träger umfaßt: eine Grundplatte (3) und einen separaten, erhöhten Abschnitt (4), der an seinem Umfang eine endlose, stehende Wand (5) mit einer kreisförmigen Grundform definiert, um die das rohrförmige Element (2) gewunden werden kann, wobei der erhöhte Abschnitt (4) auf drehbare Weise mit der Grundplatte (3) verbunden ist, indem er in einer entsprechenden kreisförmigen Öffnung (19) der Grundplatte (3) aufgenommen ist; Einfassungselemente (6), um zu verhindern, daß das rohrförmige Element (2)) sich von der stehenden Wand (5) wegbewegt, und die an dem Ende der stehenden Wand (5) nach außen vorstehen, das von der Grundplatte (3) weggedreht ist; und Befestigungsmittel (8, 9), für eines der Enden des rohrförmigen Elements an der Grundplatte (3) angeordnet und für das andere Ende, an dem erhöhten Abschnitt (4) angeordnet, wobei der erhöhte Abschnitt (4) einen Flansch (17) an dem Ende der stehenden Wand (5) umfaßt, das nahe der Grundplatte (3) liegt, **dadurch gekennzeichnet, daß** die Grundplatte (3) doppelschichtig ist und zwei Abschnitte umfaßt, wobei die kreisförmige Öffnung (19) in einem ihrer Abschnitte angeordnet ist und der Flansch (17) zwischen den beiden Abschnitten aufgenommen ist.

2. Träger zum Verpacken nach Anspruch 1, bei welchem die Grundplatte (3) ein doppelt gefalteter Bogen ist.

3. Träger zum Verpacken nach Anspruch 1, bei welchem die Einfassungselemente (6) durch Elemente gebildet sind, die aus der stehenden Wand ausgeschnitten sind.

4. Träger zum Verpacken nach Anspruch 1, bei welchem der erhöhte Abschnitt (4) einen Drehplattenabschnitt (10) umfaßt, der an der stehenden Wand (5) angrenzt.

5. Träger zum Verpacken nach Anspruch 1, wobei die Befestigungsmittel (8, 9) derart in einem Abstand von der stehenden Wand (5) ausgebildet sind, daß die Enden des rohrförmigen Elements (2), die mit ihnen verbunden sind, sich frei über eine Länge erstrecken können.

6. Träger zum Verpacken nach Anspruch 1, hergestellt aus Karton.

7. Träger zum Verpacken nach Anspruch 1, hergestellt aus einem blasgeformten dünnen Bogen aus Kunststoffmaterial.

## Revendications

1. Support d'emballage pour éléments tubulaires tels que des cathéters, ledit support comprenant : une plaque de base (3) et une section surélevée séparée (4), laquelle définit sur sa circonférence une paroi verticale sans bords (5) avec une forme circulaire de base autour de laquelle peut s'enrouler l'élément tubulaire (2), ladite section surélevée (4) étant reliée à la plaque de base (3) de manière à pouvoir effectuer une rotation, en étant reçue dans une ouverture circulaire correspondante (19) de la plaque de base (3) ; des éléments d'enfermement (6) pour empêcher l'élément tubulaire (2) de s'écarter de la paroi verticale (5), faisant saillie vers l'extérieur à l'extrémité de la paroi verticale (5) qui est détournée de la plaque de base (3) ; et des moyens de fixation (8, 9) pour l'une des extrémités de l'élément tubulaire disposée sur la plaque de base (3) et pour l'autre extrémité disposée sur la section surélevée (4), dans lequel la section surélevée (4) comprend une bride (17) à l'extrémité de la paroi verticale (5) située à proximité de la plaque de base (3), **caractérisé en ce que** la plaque de base (3) est constituée de deux couches, comprenant deux sections, dans lesquelles l'ouverture circulaire (19) a été disposée dans l'une des sections de celle-ci et la bride (17) est reçue entre les deux sections.

2. Support d'emballage selon la revendication 1, dans lequel la plaque de base (3) est une feuille pliée en double.

3. Support d'emballage selon la revendication 1, dans lequel les éléments d'enfermement (6) sont formés par des éléments découpés dans la paroi verticale.

4. Support d'emballage selon la revendication 1, dans lequel la section surélevée (4) comprend une section de plaque plate (10) contiguë à la paroi verticale (5).

5. Support d'emballage selon la revendication 1, dans lequel les moyens de fixation (8, 9) sont formés à une certaine distance de la paroi verticale (5) de telle sorte que les extrémités de l'élément tubulaire (2) qui leur sont reliées puissent s'étendre librement sur une certaine distance.

6. Support d'emballage selon la revendication 1, réalisé en carton.

7. Support d'emballage selon la revendication 1, réalisé dans une fine feuille de matière plastique soufflée.
